(19) Europäisches Patentamt
European Patent Office
Office européen des brevets

(11) **EP 3 061 743 A1**

(12) **EUROPÄISCHE PATENTANMELDUNG**

(43) Veröffentlichungstag:
**31.08.2016 Patentblatt 2016/35**

(51) Int Cl.:
***C07C 209/48*** *(2006.01)*

(21) Anmeldenummer: **15156476.2**

(22) Anmeldetag: **25.02.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **BASF SE**
**67056 Ludwigshafen am Rhein (DE)**

(72) Erfinder:
• **Wigbers, Christof Wilhelm, Dr.**
**35423 Lich (DE)**

• **Mägerlein, Wolfgang, Dr.**
**67117 Limburgerhof (DE)**
• **Krug, Thomas**
**67550 Worms (DE)**
• **Melder, Johann-Peter, Dr.**
**67459 Böhl-Iggelheim (DE)**

(74) Vertreter: **Ellwanger, Arndt et al**
**Ellwanger & Baier**
**Patentanwälte Partnerschaftsgesellschaft**
**Friedrichsplatz 9**
**68165 Mannheim (DE)**

(54) **Verfahren zur Herstellung von primären Aminen unter Verwendung eines Nickel-Festbettkatalysators**

(57) Die vorliegende Erfindung betrifft ein Verfahren zur Herstellung von primären Aminen, wobei ein Nitril in Gegenwart eines Nickel-Festbettkatalysators sowie von Ammoniak unter Erhalt des entsprechenden primären Amins hydriert wird. Die Hydrierung wird in einer Vorrichtung (V1), beispielsweise in einem Reaktor, durchgeführt. In diese Vorrichtung (V1) wird insbesondere während des Hydriervorgangs wiederkehrend oder kontinuierlich mindestens eine Verbindung (I) zugegeben, die auf Alkalimetallen, Erdalkalimetallen und/oder seltenen Erdmetallen basiert, beispielsweise wird als Verbindung (I) eine wässrige Lithiumhydroxid-Lösung zugegeben.

EP 3 061 743 A1

**Beschreibung**

Die vorliegende Erfindung betrifft ein Verfahren zur Herstellung von primären Aminen, wobei ein Nitril in Gegenwart eines Nickel-Festbettkatalysators sowie von Ammoniak unter Erhalt des entsprechenden primären Amins hydriert wird. Die Hydrierung wird in einer Vorrichtung (V1), beispielsweise in einem Reaktor, durchgeführt. In diese Vorrichtung (V1) wird insbesondere während des Hydriervorgangs wiederkehrend oder kontinuierlich mindestens eine Verbindung (I) zugegeben, die auf Alkalimetallen, Erdalkalimetallen und/oder seltenen Erdmetallen basiert, beispielsweise wird als Verbindung (I) eine wässrige Lithiumhydroxid-Lösung zugegeben:

$$R\text{-}CN + 2\,H_2 \xrightarrow[\text{[Katalysator]}]{\text{LiOH}} R\text{-}CH_2\text{-}NH_2$$

Als erfindungsgemäße Katalysatoren werden Nickel-Festbettkatalysatoren verwendet, wie sie auch für die hydrierende Aminierung von primären oder sekundären Alkoholen, Aldehyden und/oder Ketonen mit Wasserstoff und einer Stickstoffverbindung, ausgewählt aus der Gruppe Ammoniak, primäre und sekundäre Amine, zu primäre Aminen verwendet werden:

$$R\text{-}CH_2\text{-}OH + NH_3 + H_2 \xrightarrow[\text{[Katalysator]}]{} R\text{-}CH_2\text{-}NH_2 + H_2O$$

Ein Übersichtsartikel von Th. Maschmeyer et al. zeigt in Schema 9 auf Seite 1042 die große Ähnlichkeit zwischen hydrierender Aminierung und Nitrilhydrierung, die durch die gemeinsame Iminzwischenstufe zustande kommt. Aus Schema 9 geht auch die Bildung von sekundären und tertiären Aminen als Nebenprodukte hervor (Th. Maschmeyer et al: The Reductive Amination of Aldehydes and Ketones and the Hydrogenation of Nitriles: Mechanistic Aspects and Selectivity Control. Adv. Synth. Catal. 344 (2002), Nr. 10, 1047).

Die zum Beispiel in EP 963975 A1, EP 696572 B1 und WO 2011/1067199 A1 beschrieben, zum Stand der Technik gehörenden Nickel-Festbettkatalysatoren sind also nicht nur für die hydrierende Aminierung von Alkoholen, Aldehyden und Ketonen, sondern auch für die erfindungsgemäße Nitrilhydrierung geeignet.

Bei der katalytischen Hydrierung von Nitrilen zu primären Aminen in Gegenwart von Katalysatoren, die als katalytisch aktive Komponente hauptsächlich Cobaltverbindungen enthalten, kann die unerwünschte Bildung von sekundären und tertiären Aminen durch den Zusatz von Ammoniak oder Alkalihydroxiden weitgehend, aber nicht vollständig unterdrückt werden.

EP-A 913 388 beschreibt ein verbessertes Verfahren zur Hydrierung von Nitrilen zu primären Aminen, insbesondere von Dimethylaminopropionitril (DMAPN) zu Dimethylaminopropylamin (DMAPA). Hydriert wird in Gegenwart von suspendierten Kobaltschwamm-Katalysatoren wie Raney-Kobalt, Lithiumverbindungen und von Wasser ohne Zusatz von Ammoniak. Der Raney-Kobalt-Katalysator wird vor der Hydrierung mit 0,1 bis 100 mmol Lithiumhydroxid pro Gramm Kobaltschwamm-Katalysator behandelt. Nachteilig an der Fahrweise von EP-A 913 388 ist, dass die Abtrennung von Suspensionskatalysatoren aus Hydrierausträgen und ihre Rückführung in die Hydrierung oft mit Schwierigkeiten behaftet ist. In EP-A 913 388 werden neben den beschriebenen Raney-Kobalt-Katalysatoren in den Vergleichsbeispielen auch Raney-Nickel-Katalysatoren eingesetzt. Aufgrund der speziellen Herstellungsweise dieser Raney-Katalysatoren weisen diese eine sehr hohe Oberfläche auf.

WO 2003/070 688 beschreibt die Hydrierung von Nitrilen zu primären Aminen in Gegenwart von Nickel oder Kobalt-Katalysatoren, die ex situ mit Alkalicarbonaten oder Alkalihydrogencarbonaten, bevorzugt Kaliumcarbonat, vorbehandelt wurden. Unter ex situ ist dabei zu verstehen, dass der Katalysator außerhalb, insbesondere zeitlich vor der Hydrierungsreaktion von Nitril zu Amin modifiziert wurde. Als geeignete Katalysatoren werden insbesondere Raney-Nickel-Katalysatoren beschrieben, gegebenenfalls können auch Raney-Kobalt-Katalysatoren eingesetzt werden.

Nirgendwo ist jedoch beschrieben, dass die Vorbehandlung mit den Alkaliverbindungen auch während der Hydrierung kontinuierlich oder wiederkehrend erfolgen kann. Weiterhin ist kein Hinweis enthalten, dass auch Lithiumverbindungen hierfür geeignet sind.

Die der vorliegenden Erfindung zugrundeliegende Aufgabe besteht in der Bereitstellung des neuen Verfahrens zur Herstellung von primären Aminen aus den entsprechenden Nitrilen unter Verwendung eines nickelhaltigen Katalysators.

Gelöst wird die Aufgabe durch ein Verfahren zur Herstellung von primären Aminen, das dadurch gekennzeichnet ist, dass mindestens ein Nitril in einer Vorrichtung (V1) in Gegenwart eines Nickel-Festbettkatalysators und in Gegenwart von Ammoniak unter Erhalt von mindestens einem primären Amin hydriert wird, wobei in die Vorrichtung (V1) wiederkehrend oder kontinuierlich mindestens eine Verbindung (I) zugegeben wird und die Verbindung (I) mindestens eine Komponente ausgewählt aus Alkalimetall-Verbindung, Erdalkalimetall-Verbindung oder Seltene Erdmetall-Verbindung umfasst.

Durch das erfindungsgemäße Verfahren können in vorteilhafter Weise primäre Amine hergestellt werden, da Nickel-Festbettkatalysatoren eingesetzt werden. Dadurch ist auch eine Niederdruck-Hydrierung der entsprechenden Nitrile mit Wasserstoff in der Flüssigphase möglich. Weiterhin zeichnet sich das erfindungsgemäße Verfahren durch eine hohe Aktivität und Standzeit der eingesetzten Nickel-Festbettkatalysatoren aus. Aufgrund der kontinuierlichen oder zumindest wiederkehrenden Zugabe von mindestens einer Verbindung (I), insbesondere in Form von wässrigem Lithiumhydroxid, ist zudem eine hohe Ausbeute und Selektivität der gebildeten primären Amine festzustellen. Weitere Vorteile des erfindungsgemäßen Verfahrens sind darin zu sehen, dass das Verfahren auch in Gegenwart von Wasser, insbesondere unter Einsatz von wasserhaltigen Nitrilen, durchgeführt werden kann.

Ein weiterer Vorteil der vorliegenden Erfindung ist darin zu sehen, dass aufgrund der Verwendung von Nickel-Festbettkatalysatoren im erfindungsgemäßen Verfahren in der Vorrichtung (V1), insbesondere in einem Reaktor, zwischen einer Nitrilhydrierung und einer Alkoholaminierung gewechselt werden kann, ohne dass dabei der entsprechende Katalysator aus- bzw. eingebaut werden muss. Sowohl diese Wechselfahrweise als solche als auch die spezifische Alkoholaminierung bzw. die Nitrilhydrierung für sich genommen können in vorteilhafter Weise jeweils in kontinuierlicher Fahrweise durchgeführt werden. Die Anwesenheit von Ammoniak, die häufig bei Nitrilhydrierungen aufgrund eines höheren Kostenfaktors hinsichtlich Investment- und Energiekosten infolge von Abtrennung und Rückführung von Ammoniak einen unerwünschten Nachteil darstellt, ist im Fall der wechselseitig durchführbaren Alkoholaminierung von Vorteil.

Bei der Hydrierung von Nitrilen zu den entsprechenden Aminen ist es häufig erforderlich, einen hohen Umsetzungsgrad bezüglich der eingesetzten Nitrile zu erzielen, da nicht umgesetzte oder nur teilweise umgesetzte Nitrile nur schwer abzutrennen sind und in den Folgeanwendungen zu unerwünschten Eigenschaften, wie Geruch und Verfärbung, führen können.

Der Vorteil der vorliegenden Erfindung besteht somit auch darin, dass das erfindungsgemäße Verfahren die Hydrierung von Nitrilen bei hohen Umsätzen in hoher Selektivität und Ausbeute über lange Reaktionszeiten ermöglicht. Außerdem wird die Bildung von unerwünschten Nebenprodukten wie sekundären Aminen noch weiter verringert.

Dadurch ist es möglich, die Hydrierung unter milderen Reaktionsbedingungen, insbesondere bei niedrigerem Druck und/oder bei niedrigerer Temperatur durchzuführen.

Somit ermöglicht die vorliegende Erfindung ein wirtschaftliches Verfahren zur Hydrierung. Insbesondere wird die Bildung von sekundären und tertiären Aminen, wie sie beispielsweise durch Reaktion von gebildetem Amin mit partiell hydriertem Nitril (Imin-Zwischenstufe) entstehen kann, verringert.

Insbesondere ermöglicht das erfindungsgemäße Verfahren die Herstellung von Isophorondiamin mit hoher Selektivität und Ausbeute. Insbesondere ist es möglich, den Gehalt an unerwünschtem Isophoronnitrilamin (IPNA) zu verringern. IPNA kann beispielsweise durch Reaktion von Isophoronnitril mit Ammoniak entstehen, welches zunächst zu Isophoronnitrilimin reagiert, das sich dann bevorzugt mit Wasserstoff zu Isophoronnitrilamin umsetzt.

Das erfindungsgemäße Verfahren dient ebenfalls bevorzugt zur Herstellung von 3-(Dimethylamino)-propylamin (DMAPA). Insbesondere ermöglicht das erfindungsgemäße Verfahren eine Verringerung des Gehalts an Bis-DMAPA. DMAPA wird als Zwischenprodukt zum Beispiel zur Herstellung von oberflächenaktiven Substanzen, Seifen, Kosmetik, Shampoos, Hygieneprodukten, Waschmitteln und Pflanzenschutzmitteln verwendet. DMAPA wird auch zur Wasserbehandlung und als Polymerisationskatalysator für PU und Epoxy eingesetzt.

Ein Vorteil des erfindungsgemäßen Verfahrens gegenüber Verfahren des Standes der Technik, die auf Raney-Kobalt- bzw. Raney-Nickel-Katalysatoren in Suspensionsfahrweise beruhen, ist auch darin zu sehen, dass solche Raney-Katalysatoren zum Einsatz in einer Festbettfahrweise wenig geeignet sind. Die Abtrennung dieser Raney-Katalysatoren ist nur unter technisch anspruchsvollen Bedingungen, wie durch Querstromfiltration möglich.

Nachfolgend wird das erfindungsgemäße Verfahren zur Herstellung von primären Aminen unter Verwendung eines Nickel-Festbettkatalysators näher definiert.

Im erfindungsgemäßen Verfahren können prinzipiell alle Nitrile, die dem Fachmann bekannt sind, eingesetzt werden. Erfindungsgemäß kann ein einziges Nitril oder aber auch ein Gemisch von zwei oder mehreren Nitrilen eingesetzt werden. Die eingesetzten Nitrile (Edukte) können dabei eine oder auch mehrere Nitrilfunktionen aufweisen. Erfindungsgemäß wird bei der Hydrierung die Nitrilfunktion der eingesetzten Nitrile in die Aminfunktion überführt.

Vorzugsweise ist das Nitril ein aliphatisches Mono-, Di- oder Trinitril, ein cycloaliphatisches Mono- oder Dinitril, ein alpha-, beta- oder omega-Aminonitril oder ein Alkoxynitril.

Weiterhin bevorzugt werden aliphatische Mono-, Di und/oder Trinitrile (linear oder verzweigt) mit 1 bis 30, insbesondere 2 bis 18 bzw. 2 bis 8 Kohlenstoffatomen oder cycloaliphatische Mono- und Dinitrile mit 6 bis 20, insbesondere 6 bis 12 Kohlenstoffatomen oder alpha-, beta- oder omega-Aminonitrile oder Alkoxynitrile mit 1 bis 30, insbesondere 2 bis 8 Kohlenstoffatomen im erfindungsgemäßen Verfahren eingesetzt.

Weiterhin können bevorzugt aromatische Nitrile mit 6 bis 18 Kohlenstoffatomen eingesetzt werden. Die oben genannten Mono-, Di- bzw. Trinitrile können ein- oder mehrfach substituiert sein.

Besonders bevorzugte Mononitrile sind Acetonitril zur Herstellung von Ethylaminen, Propionitril zur Herstellung von Propylaminen, Butyronitril zur Herstellung von Butylaminen, Lauronitril zur Herstellung von Laurylamin, Stearylnitril zur Herstellung von Stearylamin, N,N-Dimethylaminopropionitril (DMAPN) zur Herstellung von N,N-Dimethylaminopropylamin (DMAPA) und Benzonitril zur Herstellung von Benzylamin.

Besonders bevorzugte Dinitrile sind Adipodinitril (ADN) zur Herstellung von Hexamethylendiamin (HMD) und/oder 6-Aminocapronitril (ACN), 2-Methylglutarodinitril zur Herstellung von 2-Methyl-glutarodiamin, Succinonitril zur Herstellung von 1,4-Butandiamin und Korksäuredinitril zur Herstellung von Octamethylendiamin.

Besonders bevorzugte cyclische Nitrile sind Isophoronnitrilimin (IPNI) und/oder Isophoronnitril (IPN) zur Herstellung von Isophorondiamin und Isophthalodinitril zur Herstellung von meta-Xylylendiamin.

Besonders bevorzugte $\beta$-Aminonitrile sind Aminopropionitril zur Herstellung von 1,3-Diaminopropan oder Additionsprodukte von Alkylaminen, Alkyldiaminen oder Alkanolaminen an Acrylnitril. So können Additionsprodukte von Ethylendiamin und Acrylnitril zu den entsprechenden Diaminen umgesetzt werden. Beispielsweise können 3-[2-Aminoethyl]amino]propionitril zu 3-(2-Aminoethyl)-aminopropylamin und 3,3'-(Ethylendiimino)bispropionitril bzw. 3-[2-(3-Amino-propylamino)-ethyl- amino]-propionitril zu N,N'-Bis-(3-aminopropyl)-ethylendiamin umgesetzt werden.

Besonders bevorzugte $\omega$-Aminonitrile sind Aminocapronitril zur Herstellung von Hexamethylendiamin und Caprolactam.

Weitere besonders bevorzugte Nitrile sind sogenannte "Strecker-Nitrile", wie Iminodiacetonitril zur Herstellung von Diethylentriamin und Aminoacetonitril (AAN) zur Herstellung von Ethylendiamin (EDA) und Diethylentriamin (DETA).

Ein bevorzugtes Trinitril ist Trisacetonitrilamin.

In einer besonders bevorzugten Ausführungsform wird N,N-Dimethylaminopropionitril (DMAPN) zur Herstellung von N,N-Dimethylaminopropylamin (DMAPA) in das erfindungsgemäße Verfahren eingesetzt.

In einer weiteren besonders bevorzugten Ausführungsform wird Isophoronnitrilimin zur Herstellung von Isophorondiamin in das erfindungsgemäße Verfahren eingesetzt und in einer weiteren besonders bevorzugten Ausführungsform wird Adipodinitril (ADN) zur Herstellung von Hexamethylendiamin (HMD) oder zur Herstellung von 6-Aminocapronitril (6-ACN) und HMD eingesetzt.

Die für die Hydrierung eingesetzten Nitrile können 0,01 bis 10, bevorzugt 0,1 bis 8, besonders bevorzugt 0,1 bis 5, ganz besonders bevorzugt 0,1 bis 3 Gew.-% Wasser enthalten. Dieses Wasser kann beispielsweise aus dem Verfahren zur Herstellung der verwendeten Nitrile stammen.

Die Hydrierung als solche, also die Reduktion der Nitrilfunktion des eingesetzten Nitrils unter Erhalt der entsprechenden Aminfunktion, kann prinzipiell nach allen dem Fachmann bekannten Methoden durchgeführt werden.

Als Reduktionsmittel für die Hydrierung können Wasserstoff oder ein Wasserstoff enthaltendes Gas verwendet werden. Der Wasserstoff kommt im Allgemeinen technisch rein zum Einsatz. Der Wasserstoff kann auch in Form eines Wasserstoff enthaltenden Gases, d. h. in Beimengungen mit anderen Inertgasen, wie Stickstoff, Helium, Neon, Argon oder Kohlendioxid, zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Hydrierkatalysatoren, wie zum Beispiel CO, enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

Erfindungsgemäß wird die Hydrierung in einer Vorrichtung (V1) durchgeführt, in der sich neben den Edukten (Nitril sowie Reduktionsmittel) auch die weiter unten näher präzisierten weiteren Komponenten (Nickel-Festbettkatalysator, Ammoniak sowie die Verbindung (I)) befinden.

Als Vorrichtung (V1) können prinzipiell alle dem Fachmann für eine Hydrierung geeigneten Vorrichtungen verwendet werden. Bei der Vorrichtung (V1) kann es sich prinzipiell um eine einzige Vorrichtung handeln, es können aber auch zwei oder mehrere solcher Vorrichtungen parallel und/oder in Reihe geschaltet verwendet werden.

Das erfindungsgemäße Verfahren wird bevorzugt in einem Reaktor als Vorrichtung (V1) durchgeführt.

In einer bevorzugten Ausführungsform umfasst die Festbettanordnung eine Katalysatorschüttung im eigentlichen Sinn, d. h. lose, geträgerte oder ungeträgerte Formkörper, die bevorzugt in der nachfolgend beschriebenen Geometrie oder Form vorliegen.

Dazu werden die Formkörper in den Reaktor eingebracht.

Damit die Formkörper in dem Reaktor verbleiben und nicht durch diesen hindurchfallen, wird üblicherweise ein Gitterboden oder ein gas- und flüssigkeitsdurchlässiges Blech eingesetzt, auf dem die Formkörper aufliegen.

Die Formkörper können sowohl am Eingang als am Ausgang des Reaktors von einem Inertmaterial umgeben sein. Als Inertmaterial werden in der Regel Formkörper eingesetzt, die eine ähnliche Geometrie aufweisen, wie die zuvor beschriebenen Katalysatorformkörper, sich jedoch in der Reaktion inert verhalten, zum Beispiel Pallringe, Kugeln aus einem inerten Material (zum Beispiel Keramik, Steatit, Aluminium).

Die Formkörper können aber auch mit Inertmaterial durchmischt werden und als Mischung in den Reaktor eingebracht werden.

Die Katalysatorschüttung (Formkörper + gegebenenfalls Inertmaterial) weist bevorzugt eine Schüttdichte (nach EN ISO 6) im Bereich von 0,1 bis 3 kg/l, bevorzugt von 1,5 bis 2,5 kg/l und insbesondere bevorzugt 1,7 bis 2,2 kg/l auf.

Der Differenzdruck über die Schüttung beträgt bevorzugt weniger als 1000 mbar/m, bevorzugt weniger als 800 mbar/m und besonders bevorzugt weniger als 700 mbar/m. Bevorzugt liegt der Differenzdruck über die Schüttung im Bereich von 10 bis 1000 mbar/m, bevorzugt 50 bis 800 mbar/m, besonders bevorzugt 100 bis 700 mbar/m und insbesondere im Bereich von 200 bis 500 mbar/m.

Bei Rieselfahrweise (Durchflussrichtung der Flüssigkeit von oben nach unten) ergibt sich der Differenzdruck aus dem oberhalb der Katalysatorschüttung gemessenen Druck und dem unterhalb der Katalysatorschüttung gemessenen Druck.

Bei Sumpffahrweise (Durchflussrichtung der Flüssigkeit von unten nach oben) ergibt sich der Differenzdruck aus dem unterhalb der Katalysatorschüttung gemessenen Druck und dem oberhalb der Katalysatorschüttung gemessenen Druck.

Geeignete Festbettreaktoren sind beispielsweise in dem Artikel "Fixed-Bed Reactors" (Ullmann's Encyclopedia of Industrial Chemistry, veröffentlicht online: 15. Juni 2000, DOI: 10.1002/14356007.b04_199) beschrieben.

Bevorzugt wird das Verfahren in einem Schachtreaktor, Rohrbündelreaktor oder Rohrreaktor als Vorrichtung (V1) durchgeführt.

Besonders bevorzugt wird das Verfahren in einem Rohrreaktor durchgeführt.

Die Reaktoren können jeweils als einzelner Reaktor, als Serie von einzelnen Reaktoren und/oder in Form von zwei oder mehr parallelen Reaktoren eingesetzt werden.

Der spezielle Reaktoraufbau und die Durchführung der Reaktion können in Abhängigkeit von dem durchzuführenden Hydrierungsverfahren, den erforderlichen Reaktionszeiten und der Natur des eingesetzten Katalysators variieren.

Bevorzugt beträgt das Verhältnis von Höhe zu Durchmesser des Reaktors, insbesondere eines Rohrreaktors, 1 : 1 bis 500 : 1, besonders bevorzugt 2 : 1 bis 100 : 1 und insbesondere bevorzugt 5 : 1 bis 50 : 1.

Die Fließrichtung der Reaktanden (Edukte, Wasserstoff, gegebenenfalls flüssiger Ammoniak) ist in der Regel von oben nach unten bzw. von unten nach oben.

Besonders bevorzugt ist die Fließrichtung der Reaktanden (Edukte, Wasserstoff, gegebenenfalls flüssiger Ammoniak) von oben nach unten durch den Reaktor.

Die Katalysatorbelastung bei kontinuierlicher Fahrweise liegt typischerweise bei 0,01 bis 10, vorzugsweise von 0,2 bis 5, besonders bevorzugt von 0,2 bis 2 kg Edukt pro L Katalysator und Stunde.

Die Querschnittsbelastung liegt erfindungsgemäß im Bereich von 5 kg/(m$^2$ s) bis 50 kg/(m$^2$ s), bevorzugt 8 bis 25 kg/(m$^2$ s), besonders bevorzugt 10 bis 20 kg/(m$^2$ s) und insbesondere bevorzugt 12 bis 18 kg/(m$^2$s).

Die Selektivität zu primären Amin steigt mit zunehmender Querschnittsbelastung. Die Querschnittsbelastung v [kg/(m$^2$ s)] ist definiert als

$$v = \frac{Q}{A}$$

wobei Q die Durchflussmasse [kg/s] und A die Querschnittsfläche des leeren Reaktors ist [m$^2$].

Die Durchflussmasse Q ist wiederum definiert als die Summe der Massen aller zugeführten Eduktströme und Rückführströme. Wasserstoff, Kreisgase und eventuell zugeführte Inertgase werden nicht zur Berechnung der Durchflussmenge verwendet, da Wasserstoff, Kreisgase und Inertgase bei den üblichen Hydrierbedingungen in der Regel in der Gasphase vorliegen.

Um hohe Querschnittsbelastungen zu erzielen, wird bevorzugt ein Teil des Austrags (Teilaustrag) aus dem Hydrierreaktor als Rückführstrom in den Reaktor zurückgeführt (Umlaufstrom). Der Umlaufstrom kann dem Reaktor separat zugeführt werden, oder er kann besonders bevorzugt mit den zugeführten Edukten vermischt werden und zusammen mit diesen dem Reaktor wieder zugeführt werden.

Das Verhältnis von Umlaufstrom zum zugeführten Eduktstrom liegt bevorzugt im Bereich von 0,5: 1 bis 250 : 1, besonders bevorzugt im Bereich von 1 : 1 bis 200 : 1, und insbesondere bevorzugt im Bereich von 2 : 1 bis 180 : 1. Wenn hingegen in das Verfahren viel Ammoniak zugeführt wird, dann liegt das Verhältnis von Umlaufstrom zum zugeführten Eduktstrom bevorzugt im unteren Bereich der voranstehend genannten Bereiche.

In einer bevorzugten Ausführungsform der vorliegenden Erfindung wird aus der Vorrichtung (V1) ein Strom (S1) ausgeleitet, wobei der Strom (S1) das primäre Amin enthält und der Strom (S1) zumindest teilweise in die Vorrichtung (V1) rückgeleitet wird, vorzugsweise wird die Verbindung (I) zunächst in den rückgeleiteten Teil des Stromes (S1) zugegeben und als Bestandteil des Stroms (S1) in die Vorrichtung (V1) eingespeist, besonders bevorzugt wird die Verbindung (I) als wässrige Lösung in den rückgeleiteten Teil des Stroms (S1) zugegeben.

In einer weiteren bevorzugten Ausführungsform können hohe Querschnittsbelastungen erzielt werden, wenn die Reaktion in einem Reaktor mit schlanker Bauweise, insbesondere in einem Rohrreaktor mit schlanker Bauweise, durchgeführt wird.

Das Verhältnis von Höhe zu Durchmesser des Reaktors liegt deshalb, wie voranstehend beschrieben, bevorzugt im Bereich von 1 : 1 bis 500 : 1, besonders bevorzugt im Bereich von 2 : 1 bis 100 : 1 und insbesondere bevorzugt im Bereich von 5 : 1 bis 50 : 1.

Nickel-Festbettkatalysatoren als solche sind dem Fachmann bekannt. Prinzipiell können im erfindungsgemäßen Verfahren alle bekannten Nickel-Festbettkatalysatoren eingesetzt werden. Vorzugsweise wird ein einziger Nickel-Festbettkatalysator eingesetzt, gegebenenfalls können auch Gemische aus zwei oder mehreren unterschiedlichen Nickel-Festbettkatalysatoren eingesetzt werden.

Vorzugsweise werden Raney-Nickel-Katalysatoren oder auf Monoliten basierende nickelhaltige Katalysatoren nicht als Nickel-Festbettkatalysatoren angesehen, die im Rahmen der vorliegenden Erfindung eingesetzt werden.

Die im Rahmen der vorliegenden Erfindung verwendeten Nickel-Festbettkatalysatoren weisen in der Regel neben der katalytisch aktiven Masse (Aktivkomponente) als solche auch noch Komponenten wie Trägermaterialien auf, die in der Regel nicht aktiv zur katalytischen Aktivität eines entsprechenden Katalysators beitragen. Sofern der Nickel-Festbettkatalysator einen Träger umfasst, handelt es sich vorzugsweise um $ZrO_2$ als Träger. Gegebenenfalls können auch unterschiedliche Träger sowie unterschiedliche Modifikationen desselben Trägers in den Nickel-Festbettkatalysatoren der vorliegenden Erfindung eingesetzt werden. Dabei müssen die Katalysator-Träger unter den Nitril-Hydrierbedingungen gegenüber der Verbindung (I) inert sein.

Bei der katalytisch aktiven Masse handelt es sich insbesondere um Nickel, wobei neben Nickel auch noch weitere Elemente, insbesondere Metalle, als katalytisch aktive Masse enthalten sein können. In der Regel ist der Nickelanteil der katalytisch aktiven Masse mindestens 55 Gew.-% und kann bis zu 100 Gew.-% betragen (die entsprechenden Katalysatoren enthalten nur Nickel als katalytisch aktive Masse). Vorzugsweise beträgt der Nickelanteil an der katalytisch aktiven Masse mindestens 80 Gew. %, insbesondere mindestens 90 Gew.-%. Weiterhin ist es bevorzugt, dass die Obergrenze an Nickel in der katalytisch aktiven Masse in den Nickel-Festbettkatalysatoren 95 Gew.-% beträgt. Der restliche Anteil der katalytisch aktiven Masse, der nicht durch Nickel abgedeckt wird, basiert erfindungsgemäß vorzugsweise auf Kupfermengen bis zu 20 Gew.-% und/oder Promotoren (Dotierungsmittel) wie Molybdän in Mengen bis zu 3 Gew.-%.

Weiterhin ist es bevorzugt, dass der Nickel-Festbettkatalysator als katalytisch aktive Masse die Komponente i) und gegebenenfalls die Komponente ii) und/oder iii) umfasst, mit:

i) Nickel (Ni),

ii) gegebenenfalls mindestens einem weiteren Element ausgewählt aus Eisen (Fe), Kobalt (Co), Ruthenium (Ru), Rhodium (Rh), Palladium (Pd), Osmium (Os), Iridium (Ir) oder Platin (Pt), und/oder

iii) gegebenenfalls mindestens einem Promotor ausgewählt aus Chrom (Cr), Mangan (Mn), Molybdän (Mo), Titan (Ti), Zink (Zn), Zinn (Sn), Alkalimetallen, Erdalkalimetallen, Seltenen Erdmetallen oder Phosphor (P).

Unter einem Nickel-Festbettkatalystaor ist demnach in der vorliegenden Erfindung ein Katalysator zu verstehen, bei dem als katalytisch aktive Masse Nickel und gegebenenfalls weitere Elemente und gegebenenfalls Promotoren auf einen Träger aufgebracht wurden und dieser Katalysator als Festbett im Reaktor angeordnet ist.

Nach einer weiteren bevorzugten Ausführungsform der vorliegenden Erfindung beruht der Nickel-Festbettkatalysator auf einem Katalysatorvorläufer, dessen Zusammensetzung 40 bis 60 Gew.-% NiO, 10 bis 30 Gew.-% CuO, 1 bis 3 Gew.-% $MoO_3$ und 20 bis 40 Gew.-% $ZrO_2$ beträgt.

In dem erfindungsgemäßen Verfahren können somit auch Katalysatoren eingesetzt werden, die durch Reduktion von sogenannten Katalysatorvorläufern hergestellt werden.

Der Katalysatorvorläufer enthält eine aktive Masse, die eine oder mehrere katalytisch aktive Komponenten, gegebenenfalls Promotoren und ein Trägermaterial enthält.

Bei den katalytisch aktiven Komponenten (Massen) handelt es sich um sauerstoffhaltige Verbindungen der oben genannten Metalle, beispielsweise um deren Metalloxide, Carbonate bzw. Hydroxide, wie CoO, NiO, CuO und/oder deren Mischoxide.

Im Rahmen dieser Anmeldung wird der Begriff "katalytisch aktive Komponenten" für oben genannte sauerstoffhaltige Metallverbindungen verwendet, soll aber nicht implizieren, dass diese sauerstoffhaltigen Verbindungen an sich bereits katalytisch aktiv sind. Die katalytisch aktiven Komponenten weisen in der Regel erst nach erfolgter Reduktion eine katalytische Aktivität in der erfindungsgemäßen Umsetzung auf.

Die Dotierelemente (Promotoren) sind bevorzugt in Mengen von nicht mehr als 10 Gew.-%, beispielsweise von 0,1 bis 10 Gew.-%, besonders bevorzugt in Mengen von 1 bis 5 Gew.-% enthalten, jeweils bezogen auf den jeweiligen Katalysatorvorläufer.

Bevorzugte Katalysatoren enthalten 30 bis 99 Gew.-%, bevorzugt 70 bis 99 Gew.-%, besonders bevorzugt 90 bis 99 Gew.-% Nickel.

In EP-A 963 975 offenbarte Oxidgemische, die vor der Reduktion mit Wasserstoff 22 bis 40 Gew.-% $ZrO_2$, 1 bis 30 Gew.-% sauerstoffhaltige Verbindungen des Kupfers, berechnet als $CuO$, 15 bis 50 Gew.-% sauerstoffhaltige Verbindungen des Nickels, berechnet als $NiO$, wobei das molare Ni : Cu-Verhältnis größer 1 ist, 15 bis 50 Gew.-% sauerstoffhaltige Verbindungen des Kobalts, berechnet als $CoO$, 0 bis 10 Gew.-% sauerstoffhaltige Verbindungen des Aluminiums und/oder Mangans, berechnet als $Al_2O_3$ bzw. $MnO_2$, und keine sauerstoffhaltigen Verbindungen des Molybdäns enthält, beispielsweise der in loc. cit., Seite 17, offenbarte Katalysator A mit der Zusammensetzung 33 Gew.-% Zr, berechnet als $ZrO_2$, 28 Gew.-% Ni, berechnet als $NiO$, 11 Gew.-% Cu, berechnet als $CuO$ und 28 Gew.-% Co, berechnet als $CoO$, enthalten.

Als Formkörper eignen sich Formkörper mit beliebiger Geometrie bzw. Form. Bevorzugte Formen sind Tabletten, Ringe, Zylinder, Sternstränge, Wagenräder oder Kugeln, besonders bevorzugt sind Tabletten, Ringe, Zylinder, Kugeln oder Sternstränge. Ganz besonders bevorzugt ist die Strangform.

Bei Kugeln beträgt der Durchmesser der Kugelform bevorzugt 10 mm oder weniger, besonders bevorzugt 4 mm oder weniger, ganz besonders bevorzugt 3 mm oder weniger und insbesondere bevorzugt 2,5 mm oder weniger.

In einer bevorzugten Ausführungsform liegt bei Kugeln der Durchmesser der Kugelform bevorzugt im Bereich von 0,1 bis 10, besonders bevorzugt 0,5 bis 4 mm, ganz besonders bevorzugt 1 bis 3 mm und insbesondere besonders bevorzugt 1,5 bis 2,5 mm.

Bei Strängen oder Zylindern liegt das Verhältnis von Länge : Durchmesser bevorzugt im Bereich von 1 : 1 bis 20 : 1, besonders bevorzugt 1 : 1 bis 14 : 1, ganz besonders bevorzugt im Bereich von 1 : 1 bis 10 : 1 und insbesondere bevorzugt im Bereich von 1 : 2 bis 6 : 1.

Der Durchmesser der Stränge oder Zylinder beträgt bevorzugt 10 mm oder weniger, besonders bevorzugt 5 mm oder weniger, ganz besonders bevorzugt 3 mm oder weniger und insbesondere bevorzugt 2, 5 mm oder weniger.

In einer bevorzugten Ausführungsform liegt der Durchmesser der Stränge oder Zylinder vorzugsweise im Bereich von 0,1 bis 10 mm, besonders bevorzugt im Bereich von 0,5 bis 3 mm, ganz besonders bevorzugt im Bereich von 1 bis 2,5 mm und insbesondere bevorzugt im Bereich von 1,5 bis 2,5 mm.

Bei Tabletten beträgt die Höhe h der Tablette vorzugsweise 10 mm oder weniger, besonders bevorzugt 4 mm oder weniger, ganz besonders bevorzugt 3 mm oder weniger und insbesondere bevorzugt 2,5 mm oder weniger.

In einer bevorzugten Ausführungsform liegt die Höhe h der Tablette bevorzugt im Bereich von 0,1 bis 10 mm, besonders bevorzugt im Bereich von 0,5 bis 4 mm, ganz besonders bevorzugt im Bereich von 1 bis 3 mm und insbesondere bevorzugt im Bereich von 1,5 bis 2,5 mm. Das Verhältnis von Höhe h (bzw. Dicke) der Tablette zum Durchmesser D der Tablette beträgt bevorzugt 1 : 1 bis 1 : 5, besonders bevorzugt 1 : 1 bis 1 : 2,5, ganz besonders bevorzugt 1 : 1 bis 1 : 2 und insbesondere bevorzugt 1 : 1 bis 1 : 2.

Bei allen anderen Geometrien weist der Katalysatorformkörper im erfindungsgemäßen Verfahren jeweils bevorzugt einen Äquivalentdurchmesser L = 1/a' von 2 mm oder weniger, besonders bevorzugt 1 mm oder weniger, ganz besonders bevorzugt 0,7 mm oder weniger, und insbesondere bevorzugt 0,5 mm oder weniger auf, wobei a' die externe Oberfläche per Volumeneinheit ($mm_s^2/mm^3$) ist, mit:

$$a' = \frac{A_p}{V_p}$$

wobei $A_p$ die externe Oberfläche des Formkörpers ($mm_s^2$) und $V_p$ das Volumen des Formkörpers ($mm^3$) ist.

In einer bevorzugten Ausführungsform weist der Katalysatorformkörper im erfindungsgemäßen Verfahren bei allen anderen Geometrien jeweils bevorzugt einen Äquivalentdurchmesser L = 1/a' im Bereich von 0,1 bis 2 mm, besonders bevorzugt im Bereich von 0,1 bis 0,7 mm, ganz besonders bevorzugt im Bereich von 0,2 bis 0,5 mm und insbesondere bevorzugt im Bereich von 0,3 bis 0,4 mm auf.

Die Oberfläche und das Volumen des Formkörpers ergeben sich aus den geometrischen Abmessungen des Formkörpers gemäß den bekannten mathematischen Formeln.

Das Volumen kann auch nach folgender Methode berechnet werden, bei der man:

1. die innere Porosität des Formkörpers bestimmt (zum Beispiel über Messung der Wasseraufnahme in [ml/g Kat] bei Raumtemperatur und 1 bar Gesamtdruck),

2. die Verdrängung des Formkörpers beim Eintauchen in eine Flüssigkeit (zum Beispiel durch Gasverdrängung mittels Helium-Pyknometer) bestimmt und

3. die Summe beider Volumina bildet.

Die Oberfläche kann auch nach folgender Methode theoretisch berechnet werden, bei der man eine Umhüllende des Formkörpers definiert, deren Kurvenradien max. 5 $\mu$m beträgt (um nicht die innere Porenoberfläche durch "Eindringen" der Umhüllenden in die Poren mitzunehmen) und die den Formkörper möglichst innig berührt (keine Schnittfläche mit dem Träger). Anschaulich würde das einer sehr dünnen Folie entsprechen, die man um den Formkörper legt und dann von innen ein Vakuum anlegt, so dass sich die Folie möglichst eng um den Formkörper legt.

Der eingesetzte Formkörper weist bevorzugt eine Schüttdichte (nach EN ISO 6) im Bereich von 0,1 bis 3 kg/l, bevorzugt von 1,5 bis 2,5 kg/l und insbesondere bevorzugt 1,7 bis 2,2 kg/l auf.

In einer bevorzugten Ausführungsform werden die Katalysatoren in Form von Formkörpern in das erfindungsgemäße Verfahren eingesetzt, die durch Tränkung (Imprägnierung) von Trägermaterialien hergestellt werden, die oben genannte Geometrie aufweisen oder die nach der Tränkung zu Formkörpern, die die oben genannte Geometrie aufweisen, verformt werden

Als Trägermaterialien kommen beispielsweise Kohlenstoffverbindungen, wie Graphit, Ruß, Graphen, Kohlenstoff-Nanoröhrchen und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Die Tränkung der obengenannten Trägermaterialien kann nach den üblichen Verfahren erfolgen (A. B. Stiles, Catalyst Manufacture - Laboratory and Commercial Preparations, Marcel Dekker, New York, 1983), beispielsweise durch Aufbringung einer Metallsalzlösung in einer oder mehreren Tränkstufen. Als Metallsalze kommen in der Regel wasserlösliche Metallsalze, wie die Nitrate, Acetate oder Chloride der entsprechenden katalytisch aktiven Komponenten oder Dotierelemente in Betracht, wie Co-Nitrat oder Co-Chlorid. Im Anschluss wird das getränkte Trägermaterial in der Regel getrocknet und ggf. calciniert.

Die Calcinierung wird im Allgemeinen bei Temperaturen zwischen 300 und 800 °C, vorzugsweise 350 bis 600 °C, insbesondere bei 450 bis 550 °C ausgeführt.

Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Metallsalzen beaufschlagt werden soll.

Zur Aufbringung mehrerer Metallkomponenten auf das Trägermaterial, kann die Tränkung gleichzeitig mit allen Metallsalzen oder in beliebiger Reihenfolge der einzelnen Metallsalze nacheinander erfolgen.

Bevorzugt werden Trägermaterialien eingesetzt, die bereits die zuvor beschriebene bevorzugte Geometrie der Formkörper aufweisen.

Es ist jedoch auch möglich Trägermaterialien einzusetzen, die als Pulver oder Splitt vorliegen, und getränkte Trägermaterialien einer Formgebung zu unterziehen

So kann beispielsweise das imprägnierte und getrocknete bzw. calcinierte Trägermaterial konditioniert werden.

Die Konditionierung kann beispielsweise dadurch erfolgen, dass man das getränkte Trägermaterial durch Vermahlen auf eine bestimmte Korngröße einstellt. Nach der Vermahlung kann das konditionierte, getränkte Trägermaterial mit Formhilfsmitteln wie Graphit, oder Stearinsäure vermischt werden und zu Formkörpern weiterverarbeitet werden.

Gängige Verfahren der Formgebung sind beispielsweise in Ullmann's Encyclopedia Electronic Release 2000, Kapitel: "Catalysis and Catalysts", Seiten 28 - 32 und von Ertl et al. (Ertl, Knözinger, Weitkamp, Handbook of Heterogenoeous Catalysis, VCH Weinheim, 1997, Seiten 98 ff.) beschrieben.

Gängige Verfahren der Formgebung sind beispielsweise Extrusion, Tablettieren, d. h. mechanisches Verpressen oder Pelletieren, d. h. Kompaktieren durch kreisförmige und/oder rotierende Bewegungen.

Durch den Prozess der Formgebung können Formkörper mit der oben genannten Geometrie erhalten werden.

Nach der Konditionierung bzw. Formgebung erfolgt in der Regel eine Temperung. Die Temperaturen bei der Temperung entsprechen üblicherweise den Temperaturen bei der Calcinierung.

In einer bevorzugten Ausführungsform werden Formkörper in das erfindungsgemäße Verfahren eingesetzt, die durch eine gemeinsame Fällung (Mischfällung) aller ihrer Komponenten hergestellt werden und die so ausgefällten Katalysatorvorläufer einer Formgebung unterzogen werden.

Dazu wird in der Regel eine lösliche Verbindung der entsprechenden aktiven Komponente, der Dotierelemente und gegebenenfalls eine lösliche Verbindung eines Trägermaterials in einer Flüssigkeit in der Wärme und unter Rühren so lange mit einem Fällungsmittel versetzt, bis die Fällung vollständig ist.

Als Flüssigkeit wird in der Regel Wasser eingesetzt.

Als lösliche Verbindung der aktiven Komponenten kommen üblicherweise die entsprechenden Metallsalze, wie die Nitrate, Sulfate, Acetate oder Chloride, der voranstehend genannten Metalle in Betracht.

Als lösliche Verbindungen eines Trägermaterials werden in der Regel wasserlösliche Verbindungen von Ti, Al, Zr, Si etc., beispielsweise die wasserlöslichen Nitrate, Sulfate, Acetate oder Chloride dieser Elemente, verwendet.

Als lösliche Verbindungen der Dotierelemente werden in der Regel wasserlösliche Verbindungen der Dotierelemente, beispielsweise die wasserlöslichen Nitrate, Sulfate, Acetate oder Chloride dieser Elemente, eingesetzt.

In einer weiteren, bevorzugten Ausführungsform können die Formkörper durch Auffällung hergestellt werden.

Unter Auffällung wird eine Herstellmethode verstanden, bei der ein schwer lösliches oder unlösliches Trägermaterial in einer Flüssigkeit suspendiert wird und nachfolgend lösliche Verbindungen, wie lösliche Metallsalze, der entsprechenden Metalloxide zugegeben werden, welche dann durch Zugabe eines Fällungsmittels auf den suspendierten Träger aufgefällt werden (zum Beispiel beschrieben in EP-A2 1 106 600, Seite 4, und A. B. Stiles, Catalyst Manufacture, Marcel Dekker, Inc., 1983, Seite 15).

Als schwer- bzw. unlösliche Trägermaterialien kommen beispielsweise Kohlenstoffverbindungen, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Das Trägermaterial liegt in der Regel als Pulver oder Splitt vor.

Als Flüssigkeit, in der das Trägermaterial suspendiert wird, wird üblicherweise Wasser eingesetzt.

Als lösliche Verbindungen kommen die voranstehend genannten löslichen Verbindungen der aktiven Komponenten bzw. der Dotierelemente in Betracht.

Üblicherweise werden bei den Fällungsreaktionen die löslichen Verbindungen durch Zugabe eines Fällungsmittels als schwer- oder unlösliche, basische Salze gefällt.

Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.

Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können zum Beispiel bei Temperaturen von 20 bis 100 °C, besonders 30 bis 90 °C, insbesondere bei 50 bis 70 °C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d. h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im Allgemeinen bei 80 bis 200 °C, vorzugsweise 100 bis 150 °C, getrocknet und anschließend calciniert.

Die Calcinierung wird im Allgemeinen bei Temperaturen zwischen 300 und 800 °C, vorzugsweise 350 bis 600 °C, insbesondere bei 450 bis 550 °C ausgeführt.

Nach der Calcinierung werden die durch Fällungsreaktionen erhaltenen pulverförmigen Katalysatorvorläufer üblicherweise konditioniert.

Die Konditionierung kann beispielsweise dadurch erfolgen, dass man den Fällungskatalysator durch Vermahlen auf eine bestimmte Korngröße einstellt.

Nach der Vermahlung kann der durch Fällungsreaktionen erhaltene Katalysatorvorläufer mit Formhilfsmitteln wie Graphit, oder Stearinsäure vermischt werden und zu Formkörpern weiterverarbeitet werden.

Gängige Verfahren der Formgebung sind beispielsweise in Ullmann's Encyclopedia Electronic Release 2000, Kapitel: "Catalysis and Catalysts", Seiten 28 - 32, und von Ertl et al. (Ertl, Knözinger, Weitkamp, Handbook of Heterogenoeous Catalysis, VCH Weinheim, 1997, Seiten 98 ff.) beschrieben.

Gängige Verfahren der Formgebung sind beispielsweise Extrusion, Tablettieren, d. h. mechanisches Verpressen oder Pelletieren, d.h. Kompaktieren durch kreisförmige und/oder rotierende Bewegungen.

Durch den Prozess der Formgebung können Formkörper mit der oben genannten Geometrie erhalten werden.

Nach der Konditionierung bzw. Formgebung erfolgt in der Regel eine Temperung. Die Temperaturen bei der Temperung entsprechen üblicherweise den Temperaturen bei der Calcinierung.

Formkörper, die durch Tränkung oder Fällung hergestellt wurden, enthalten in der Regel die katalytisch aktiven Komponenten nach erfolgter Calcinierung in Form ihrer sauerstoffhaltigen Verbindungen, beispielsweise als Metalloxide bzw. Hydroxide, wie CoO, NiO, CuO und/oder deren Mischoxide (Katalysatorvorläufer).

Die Katalysatorvorläufer, die wie voranstehend beschrieben durch Imprägnierung oder Fällung hergestellt wurden, werden im Allgemeinen nach der Calcinierung bzw. Konditionierung reduziert. Durch die Reduktion wird der Katalysatorvorläufer in der Regel in seine katalytisch aktive Form umgewandelt.

Die Reduktion des Katalysatorvorläufers kann bei erhöhter Temperatur in einem bewegten oder unbewegten Reduktionsofen durchgeführt werden.

Als Reduktionsmittel wird üblicherweise Wasserstoff oder ein Wasserstoff enthaltendes Gas eingesetzt.

Der Wasserstoff kommt im Allgemeinen technisch rein zum Einsatz. Der Wasserstoff kann auch in Form eines Wasserstoff enthaltendem Gases, das heißt mit Beimengungen anderer Inertgase, wie Stickstoff, Helium, Neon, Argon oder Kohlendioxid zum Einsatz kommen. Der Wasserstoffstrom kann auch als Kreisgas in die Reduktion zurückgeführt werden, gegebenenfalls vermischt mit Frischwasserstoff und gegebenenfalls nach Entfernen von Wasser durch Kondensation.

Die Reduktion des Katalysatorvorläufers erfolgt bevorzugt in einem Reaktor, in dem die Formkörper als Festbett angeordnet sind. Besonders bevorzugt erfolgt die Reduktion des Katalysatorvorläufers in demselben Reaktor, in dem die nachfolgende Umsetzung der Nitrile mit Wasserstoff erfolgt.

Weiterhin kann die Reduktion des Katalysatorvorläufers in einem Wirbelschichtreaktor in der Wirbelschicht erfolgen.

Die Reduktion des Katalysatorvorläufers erfolgt in der Regel bei Reduktionstemperaturen von 50 bis 600 °C, insbesondere von 100 bis 500 °C, besonders bevorzugt von 150 bis 450 °C.

Der Wasserstoffpartialdruck beträgt in der Regel von 1 bis 300 bar, insbesondere von 1 bis 200 bar, besonders bevorzugt von 1 bis 100 bar, wobei sich die Druckangaben hier und im Folgenden auf den absolut gemessenen Druck beziehen.

Die Dauer der Reduktion beträgt bevorzugt 1 bis 20 Stunden, und besonders bevorzugt 5 bis 15 Stunden.

Während der Reduktion kann ein Lösungsmittel zugeführt werden, um entstehendes Reaktionswasser abzuführen und/oder um beispielsweise den Reaktor schneller aufheizen zu können und/oder während der Reduktion die Wärme besser abführen zu können. Das Lösungsmittel kann hierbei auch überkritisch zugeführt werden.

Als geeignete Lösungsmittel können die zuvor beschriebenen Lösungsmittel eingesetzt werden. Bevorzugte Lösungsmittel sind Wasser; Ether wie Methyltertbutylether, Ethyltertbutylether, Dioxan oder Tetrahydrofuran. Besonders bevorzugt sind Wasser oder Tetrahydrofuran. Als geeignete Lösungsmittel kommen ebenfalls geeignete Mischungen in Betracht.

Der so erhaltene Formkörper kann nach der Reduktion unter inerten Bedingungen gehandhabt werden. Bevorzugt kann der Formkörper unter einem Inertgas wie Stickstoff gehandhabt und gelagert werden oder unter einer inerten Flüssigkeit, zum Beispiel einem Alkohol, Wasser oder dem Produkt der jeweiligen Reaktion, für die der Katalysator eingesetzt wird. Gegebenenfalls muss der Katalysator vor Beginn der eigentlichen Reaktion dann von der inerten Flüssigkeit befreit werden.

Die Lagerung des Katalysators unter inerten Substanzen ermöglicht eine unkomplizierte und ungefährliche Handhabung und Lagerung des Formkörpers.

Der Formkörper kann nach der Reduktion aber auch mit einem Sauerstoff enthaltenden Gasstrom wie Luft oder einem Gemisch von Luft mit Stickstoff in Kontakt gebracht werden.

Dadurch wird ein passivierter Formkörper erhalten. Der passivierte Formkörper weist im Allgemeinen eine schützende Oxidschicht auf. Durch diese schützende Oxidschicht wird die Handhabung und Lagerung des Katalysators vereinfacht, so dass beispielsweise der Einbau des passivierten Formkörpers in den Reaktor vereinfacht wird. Ein passivierter Formkörper wird bevorzugt vor dem Inkontaktbringen mit den Edukten wie oben beschrieben durch Behandlung des passivierten Katalysators mit Wasserstoff oder einem Wasserstoff enthaltenden Gas reduziert. Die Reduktionsbedingungen entsprechen im Allgemeinen den Reduktionsbedingungen, die bei der Reduktion der Katalysatorvorläufer angewandt werden. Durch die Aktivierung wird in der Regel die schützende Passivierungsschicht aufgehoben.

In die Vorrichtung (V1) - also die Hydrierung - wird erfindungsgemäß wiederkehrend oder kontinuierlich mindestens eine Verbindung (I) zugegeben. Die Verbindung (I) umfasst mindestens eine Komponente ausgewählt aus einer Alkalimetall-Verbindung, Erdalkalimetall-Verbindung oder Seltenen Erdmetall-Verbindung. Die Verbindung (I) als solche ist dem Fachmann bekannt. Vorzugsweise wird eine Verbindung (I) wiederkehrend oder kontinuierlich in die Vorrichtung (V1) zugegeben, gegebenenfalls können auch Gemische aus zwei oder mehreren Verbindungen (I) zugegeben werden. Weiterhin ist es möglich, dass sich bereits vor Beginn der Hydrierung mindestens eine Verbindung (I) in der Vorrichtung (V1) befindet, vorzugsweise wird die Verbindung (I) aber erst nach Beginn der Hydrierung in das erfindungsgemäße Verfahren zugegeben.

Die Zugabe von mindestens einer Verbindung (I) in die Vorrichtung (V1) kann wie vorstehend bereits ausgeführt wiederkehrend oder kontinuierlich erfolgen. Dabei kann die Verbindung (I) in flüssiger oder fester Form zugegeben werden. Dabei ist auch festzuhalten, dass die Verbindung (I) nicht direkt in die Vorrichtung (V1) zugegeben werden muss. Die Verbindung (I) kann zunächst auch in einer anderen Vorrichtung, beispielsweise in einer Kontaktvorrichtung (V2), einer oder mehreren der an der Hydrierung beteiligten Komponenten zugegeben werden. Aus dieser anderen Vorrichtung wird die Verbindung (I) in die Vorrichtung (V1) geführt (mittelbare Zugabe die Verbindung (I) nach (V1)). Das (Über-)Führen oder (Über-)Leiten der Verbindung (I) aus der anderen Vorrichtung in die Vorrichtung (V1) erfolgt nach den dem Fachmann bekannten Methoden, beispielsweise unter Verwendung von Pumpen. Insbesondere kann die Verbindung (I) wie vorstehend bereits ausgeführt einem rückgeleiteten Teil des Stroms (S1) zugegeben werden.

Unter einer "kontinuierlichen Zugabe" der Verbindung (I) wird im Rahmen der vorliegenden Erfindung verstanden, dass die entsprechende Zugabe über einen längeren Zeitraum, vorzugsweise über mindestens 50 %, mehr bevorzugt über mindestens 70 %, noch mehr bevorzugt über mindestens 90 %, insbesondere über die gesamte Reaktionsdauer erfolgt. Vorzugsweise wird die kontinuierliche Zugabe so durchgeführt, dass die entsprechende Vorrichtung zum Eintrag (Zugabe) der Verbindung (I) (zum Beispiel eine Zellenradschleuse) über die vorgenannten Zeiträume in Betrieb ist.

Unter einer "wiederkehrenden Zugabe" der Verbindung (I) wird im Rahmen der vorliegenden Erfindung verstanden, dass die entsprechende Zugabe in regelmäßigen oder unregelmäßigen Zeitabständen erfolgt. Vorzugsweise wird die entsprechende Zugabe durch das Eintreten einer weiter unten beschriebenen Zugabebedingung veranlasst, insbesondere im Zusammenhang mit der Konzentration der Verbindung (I) im Austrag aus der Vorrichtung (V1). Die zeitlichen Abstände zwischen den einzelnen Zugaben betragen mindestens 1 h, vorzugsweise mindestens einen Tag. Im Rahmen der vorliegenden Erfindung werden unter dem Begriff "wiederkehrend" weiterhin mindestens zwei, beispielsweise 3, 4, 5, 10 oder auch 100 einzelne Zugaben verstanden. Die konkrete Anzahl der Einzelzugaben hängt von der Betriebsdauer ab. Diese geht idealerweise gegen Unendlich.

In anderen Worten ausgedrückt wird unter einer wiederkehrenden Zugabe der Verbindung (I) im Rahmen der vorliegenden Erfindung die zeitlich voneinander abgegrenzte Zugabe mehrerer Chargen an Verbindung (I) verstanden. Die Zugabe einer einzelnen Charge kann von mehreren Sekunden bis zu mehreren Minuten dauern, gegebenenfalls sind auch etwas längere Zeiträume denkbar. Erfindungsgemäß ist der zeitliche Abstand zwischen der jeweiligen Zugabe einer einzelnen Charge mindestens zehnmal so groß wie die Dauer der Zugabe der entsprechenden Charge. Gegebenenfalls kann im Rahmen der vorliegenden Erfindung die Ausführungsform einer "wiederkehrenden Zugabe" mit der Ausführungsform einer "kontinuierlichen Zugabe" kombiniert werden.

Die genaue Dosierung der wiederkehrenden oder kontinuierlichen Zugabe von mindestens einer Verbindung (I) wird nachfolgend anhand der Zugabe von Lithiumhydroxid exemplarisch verdeutlicht. Die Ausführungen gelten aber auch sinngemäß für sämtliche andere Verbindungen, die unter die Definition der Verbindung (I) der vorliegenden Erfindung fallen.

Die Lithium-Konzentration im Austrag sollte einen gewissen Schwellenwert nicht unterschreiten. Das Unterschreiten ist ein Indikator, dass Lithium aus dem Katalysator ausgelaugt wurde.

Die Lithium-Konzentration im Austrag sollte auch nicht zu hoch sein, da ansonsten der Katalysator seine Aktivität verliert (Vergiftung).

Die Dosierung kann kontinuierlich oder wiederkehrend (periodisch) erfolgen. Wesentlich ist, dass die Lithium-Konzentration im Austrag/Reaktionsgemisch nicht so hoch wird, dass es zu Ablagerungen kommt, aber so hoch ist, dass die Konzentration auf dem Katalysator erhalten bleibt.

Insbesondere gelten an der Zudosierungsstelle noch besondere Anforderungen:

- Die Lithium-Konzentration der zudosierten Lösung darf nicht zu hoch sein, da es sonst zu Ablagerungen/Verstopfungen im Reaktor kommt.

- Die Einspeisung sollte an einer Stelle des Reaktors vorgenommen werden, an der eine hohe Strömungsgeschwindigkeit herrscht, damit das LiOH sich gut mit dem Reaktionsgemisch vermischt. LiOH hat eine geringe Löslichkeit in Wasser und in den meisten organischen Lösungsmitteln.

Als Verbindungen (I) der Alkalimetalle kommen Li-, Na-, K-, Rb-, Cs-Verbindungen, als Verbindungen (I) der Erdalkalimetalle kommen Mg-, Ca-, Sr- und Ba-Verbindungen und als Verbindungen (I) der Seltenen Erdmetalle La-, Ce-, Pr- und Nd-Verbindungen in Frage.

Bevorzugte Verbindungen (I) der Alkalimetalle sind Lithium-Verbindungen, Kalium-Verbindungen und Caesium-Verbindungen, der Erdalkalimetalle MagnesiumVerbindungen und Calcium-Verbindungen und der Seltenen Erdmetalle Lanthan-Verbindungen und Cer-Verbindungen.

Weiterhin ist es bevorzugt, dass die Verbindung (I) in Form eines Oxids, eines Hydroxids und/oder eines Salzes eingesetzt wird und/oder dass die Verbindung (I) als wässrige Lösung eingesetzt wird. Als Salze kommen z.B. Nitrate, Carbonate, Hydrogencarbonate, Phosphate, Carboxylate wie z.B. Formiate und Acetate in Frage. Bevorzugt sind Hydroxide, die im Rahmen der vorliegenden Erfindung nicht unter den Begriff "Salze" fallen.

Weiterhin ist es bevorzugt, dass eine wässrige Lösung eines Oxids, eines Hydroxids oder eines Salzes des Lithiums, Kaliums, Caesiums, Magnesiums, Calciums, Lanthans und/oder Cers verwendet wird.

Ganz besonders bevorzugt sind wässrige Lösungen von Verbindungen des Lithiums wie zum Beispiel Lithiumhydroxid, Lithiumcarbonat, Lithiumphosphat, Lithiumnitrat, Lithiumformiat, Lithiumacetat. Bevorzugt ist hierbei Lithiumhydroxid.

Als Lösungsmittel für Alkali-, Erdalkali- und Seltene Erdverbindungen können grundsätzlich organische Lösungsmittel, Wasser oder Gemische dieser Lösungsmittel mit Wasser verwendet werden. Bevorzugt werden die Lösungsmittel, die auch als Lösungsmittel für die eingesetzten Nitrile weiter unten genannt sind. Sie müssen unter den Hydrierbedingungen stabil sein und die Alkali-, Erdalkali- und Seltenen Erdverbindungen in ausreichendem Maße lösen.

Bevorzugt sind daher $C_1$- bis $C_4$-Alkohole, Wasser oder Gemische dieser Verbindungen. Ganz besonders bevorzugt ist Wasser.

Erfindungsgemäß wird die Nitrilhydrierung auch in Gegenwart von Ammoniak (auch in der Vorrichtung (V1)) durchgeführt. Bei dem erfindungsgemäßen Verfahren zur Herstellung von Aminen durch Reduktion von Nitrilen erfolgt die Hydrierung unter Zusatz von Ammoniak. Das Molverhältnis von Ammoniak zu Nitrilgruppe liegt beispielsweise im Bereich von 0,5 : 1 bis 100 : 1, bevorzugt im Bereich von 2 : 1 bis 20 : 1.

Weiterhin ist es erfindungsgemäß bevorzugt, dass

i) das zur Hydrierung eingesetzte Nitril 0,01 bis 10 Gew.-% Wasser enthält, und/oder

ii) die Verbindung (I) in die Vorrichtung (V1) zugegeben wird, sobald der Anteil an als Nebenprodukt gebildetem sekundären Amin größer ist als 0,5 GC-Fl%, und/oder

iii) in die Vorrichtung (V1) dem Hydriergemisch über die Zugabe der Verbindung (I) 0,01 bis 500 ppm Alkali-, Erdalkali- und/oder Seltene Erdmetall-Verbindung, bezogen auf g-Atome Nitril in der Vorrichtung (V1), zugegeben werden, vorzugsweise werden in die Vorrichtung (V1) dem Hydriergemisch über die Zugabe der Verbindung (I) 0,01 bis 500 ppm Alkali-, Erdalkali- und/oder Seltene Erdmetall-Verbindung, bezogen auf g-Atome Nitril in der Vorrichtung (V1), zugegeben, sobald der Anteil an als Nebenprodukt gebildetem sekundären Amin in der Vorrichtung (V1) größer ist als 0,5 GC-Fl%, bei einer Katalysatorbelastung von 0,01 bis 10 kg Edukt pro L Katalysator und Stunde.

Verfahren zur Bestimmung der GC-Fl% (GC-Flächenprozent) sind dem Fachmann als solche bekannt. Vorzugsweise werden die GC-Fl% mit folgenden Parametern bestimmt: GC-Säule: 60 m CP Voltamine/WCOT fused Silica 0,32; Temperaturprogramm: 50 °C - 10 min. - 15 °C/min - 240 ° - 30 min.

Die Hydrierung kann erfindungsgemäß diskontinuierlich, halbkontinuierlich oder kontinuierlich durchgeführt werden. Vorzugsweise wird die (Nitril-)Hydrierung kontinuierlich durchgeführt.

Die Hydrierung wird in der Regel bei einem Druck von 1 bis 300 bar, insbesondere von 5 bis 150 bar, bevorzugt von 10 bis 100 bar und besonders bevorzugt von 15 bis 60 bar durchgeführt. Ganz besonders bevorzugt wird die Hydrierung bei einem Druck von weniger als 60 bar als Niederdruckverfahren ausgeführt

Die Temperatur liegt in der Regel in einem Bereich von 25 bis 300 °C, insbesondere von 50 bis 200 °C, bevorzugt von 70 bis 150 °C, besonders bevorzugt von 80 bis 140 °C.

Das molare Verhältnis von Wasserstoff zu eingesetztem Nitril beträgt in der Regel 2 : 1 bis 25 : 1, vorzugsweise 2 : 1 bis 10 : 1. Überschüssiger Wasserstoff kann als Kreisgas in die Reaktion zurückgeführt werden.

Dabei werden die Reaktionsbedingungen bevorzugt so gewählt, dass die eingesetzten Nitrile und zugegebenen Flüssigkeiten in der Regel in der Flüssigphase vorliegen und nur der eingesetzte Wasserstoff bzw. Inertgase unter den genannten Reaktionsbedingungen in der Gasphase vorliegen.

Das Ergebnis der Hydrierung kann, vor allem bei kontinuierlicher Durchführung, analytisch, zum Beispiel gaschromatographisch, verfolgt werden. Falls die Analyse ein Absinken von Nitril-Umsatz und/oder Selektivität an primärem Amin offenbart, kann dieses Absinken vorzugsweise durch Zugabe der genannten Lösungen rückgängig gemacht werden.

Die Hydrierung kann in Substanz oder in einer Flüssigkeit durchgeführt werden.

Geeignete Lösungsmittel sind beispielsweise $C_1$- bis $C_4$-Alkohole, wie Methanol oder Ethanol, $C_4$- bis $C_{12}$-Dialkylether, wie Diethylether oder tert-Butylmethylether, oder cyclische $C_4$- bis $C_{12}$-Ether, wie Tetrahydrofuran oder Dioxan, oder Kohlenwasserstoffe, wie Pentan, Hexan, Heptan, Octan, Cyclohexan. Geeignete Flüssigkeiten können auch Mischungen der vorstehend genannten Flüssigkeiten sein. In einer bevorzugten Ausführungsform ist das Lösungsmittel ein Produkt der Hydrierung.

Die Aufarbeitung der Hydrierausträge erfolgt vorzugsweise destillativ. Dabei werden die zugesetzten Alkali-, Erdalkali- und/oder Seltenen Erdverbindungen als Sumpfprodukte abgetrennt

Weiterhin ist es erfindungsgemäß bevorzugt, dass die Vorrichtung (I) ein Reaktor ist, vorzugsweise beträgt die Querschnittsbelastung 5 bis 50 kg Durchflussmasse pro $m^2$ Querschnittsfläche des Reaktors und Sekunde.

Weiterhin ist es erfindungsgemäß bevorzugt, dass in der Vorrichtung (V1) unter Verwendung des gleichen Nickel-Festbettkatalysators wechselseitig eine Alkoholaminierung und eine Nitrilhydrierung durchgeführt werden kann.

Insbesondere ist es bevorzugt, dass beim Wechseln von Nitrilhydrierung zu Alkoholaminierung oder beim umgekehrten Wechsel der Nickel-Festbettkatalysator nicht eingebaut und/oder ausgebaut werden muss. Die Alkoholaminierung ist beispielsweise in der EP 963975 A1, der EP 696572 B1 und der WO 2011/067199 A1 beschrieben.

Nachfolgend wird die vorliegende Erfindung anhand von Beispielen verdeutlicht.

Beispiel 1: Hydrierung von Dimethylaminopropionitril (DMAPN) zu Dimethylaminopropylamin (DMAPA) in Gegenwart eines Nickel-Festbettkatalysators sowie von Ammoniak unter wiederkehrender Zuführung von wässriger Lithiumhydroxid-Lösung

a) Katalysatoraktivierung

Als Katalysator-Vorläufer wird ein überwiegend Nickel enthaltendes Oxidgemisch der Zusammensetzung 50 Gew.-% NiO, 17 Gew.-% CuO, 1,5 Gew.-% $MoO_3$ und 31,5 Gew.-% $ZrO_2$ verwendet. 500 ml (766,2 g) dieses Vorläufers (3 x 3 mm Tabletten) werden in den Hydrierreaktor eingefüllt und in 12 Stunden unter Zufuhr von 200 NL Wasserstoff pro Stunde auf 280 °C aufgeheizt, 12 Stunden bei dieser Temperatur unter Zuleiten von 25 NL Wasserstoff pro Stunde gehalten und unter Wasserstoff abgekühlt.

b) Hydrierung von DMAPN

Die Hydrierung wird mit dem vorstehend beschriebenen Katalysator als Festbettkatalysator in einem Rohrreaktor aus Edelstahl (Innenrohr 1.4571) in Rieselfahrweise durchgeführt, der eine Höhe von einem Meter und einen Durchmesser von 0,6 cm besitzt. Der Reaktionsaustrag der Hydrierung wird gekühlt, entspannt, zum Teil ausgeschleust und zum Teil in den Reaktor zurückgeführt.
Als Einsatzstoff für die Hydrierung wird technisches DMAPN verwendet, das Dimethylamin und Wasser enthält. Der Wassergehalt beträgt laut GC-Analyse 0,3 % bis 3,6 Flächen-%, der Gehalt an Dimethylamin 1,4 bis 2,5 Flächen-% (GC-Säule: 60 m CP Volamine/WCOT fused Silica 0,32; Temperaturprogramm: 50 °C - 10 min-15 °C/min. - 240 °C - 30 min).
Die Hydrierung wird insgesamt rund 1500 Stunden lang bei 90 bis 120 °C und 50 bis 85 bar durchgeführt. Die Katalysatorbelastung beträgt während der gesamten Laufzeit 1 bis 1,1 kg DMAPN pro Liter Katalysator und Stunde, die Querschnittsbelastung 41 bis 42 kg/(m$^2$s).
Gestartet wird die Hydrierung durch Zupumpen von 200 ml rohem DMAPA in den Reaktor. Dann wird pro Stunde 18,8 bis 20,5 g DMAPN zugeleitet. Rückgeführt in den Reaktor werden 795 bis 834 g des Hydrieraustrags pro Stunde.
Das Molverhältnis von Ammoniak zu DMAPN während der Laufzeit beträgt 15.
Während der ersten rund 650 Stunden steigt die DMAPA-Ausbeute von 90 % auf > 99,5 %, die Bis-DMAPA-Ausbeute sinkt gleichzeitig von rund 10 % auf unter 1 %. Die DMAPN-Umsätze betragen während dieser Zeit > 99,5 % (GC-Analyse auf GC-Säule: 60 m CP Voltamine/WCOT fused Silica 0,32; Temperaturprogramm: 50 °C - 10 min - 15 °C/min - 240 °C - 30 min)
Während der restlichen Laufzeit von rund 850 Stunden beträgt die DMAPA-Ausbeute > 99,3 %, die Bis-DMAPA-Ausbeute < 0,5 % und der DMAPN-Umsatz > 99,5 %.

**Patentansprüche**

1. Verfahren zur Herstellung von primären Aminen, **dadurch gekennzeichnet, dass** mindestens ein Nitril in einer Vorrichtung (V1) in Gegenwart eines Nickel-Festbettkatalysators und in Gegenwart von Ammoniak unter Erhalt von mindestens einem primären Amin hydriert wird, wobei in die Vorrichtung (V1) wiederkehrend oder kontinuierlich mindestens eine Verbindung (I) zugegeben wird und die Verbindung (I) mindestens eine Komponente ausgewählt aus Alkalimetall-Verbindung, Erdalkalimetall-Verbindung oder Seltene Erdmetall-Verbindung umfasst.

2. Verfahren gemäß Anspruch 1, **dadurch gekennzeichnet, dass** die Verbindung (I) in Form eines Oxids, eines Hydroxids und/oder eines Salzes eingesetzt wird und/oder dass die Verbindung (I) als wässrige Lösung eingesetzt wird.

3. Verfahren gemäß Anspruch 2, **dadurch gekennzeichnet, dass** eine wässrige Lösung eines Oxids, eines Hydroxids oder eines Salzes des Lithiums, Kaliums, Caesiums, Magnesiums, Calciums, Lanthans und/oder Cers verwendet wird.

4. Verfahren gemäß einem der Ansprüche 1 bis 3, **dadurch gekennzeichnet, dass**

   i) das zur Hydrierung eingesetzte Nitril 0,01 bis 10 Gew.-% Wasser enthält, und/oder
   ii) die Verbindung (I) in die Vorrichtung (V1) zugegeben wird, sobald der Anteil an als Nebenprodukt gebildetem sekundären Amin größer ist als 0,5 GC-Fl%, und/oder
   iii) in die Vorrichtung (V1) dem Hydriergemisch über die Zugabe der Verbindung (I) 0,01 bis 500 ppm Alkali-, Erdalkali- und/oder Seltene Erdmetall-Verbindung, bezogen auf g-Atome Nitril in der Vorrichtung (V1), zugegeben werden, vorzugsweise werden in die Vorrichtung (V1) dem Hydriergemisch über die Zugabe der Verbindung (I) 0,01 bis 500 ppm Alkali-, Erdalkali- und/oder Seltene Erdmetall-Verbindung, bezogen auf g-Atome Nitril in der Vorrichtung (V1), zugegeben, sobald der Anteil an als Nebenprodukt gebildetem sekundären Amin in der Vorrichtung (V1) größer ist als 0,5 GC-Fl%, bei einer Katalysatorbelastung von 0,01 bis 10 kg Edukt pro L Katalysator und Stunde.

**5.** Verfahren gemäß einem der Ansprüche 1 bis 4, **dadurch gekennzeichnet, dass** die Vorrichtung (I) ein Reaktor ist, vorzugsweise beträgt die Querschnittsbelastung 5 bis 50 kg Durchflussmasse pro m$^2$ Querschnittsfläche des Reaktors und Sekunde.

**6.** Verfahren gemäß einem der Ansprüche 1 bis 5, **dadurch gekennzeichnet, dass** die Hydrierung bei Drucken von 1 bis 300 bar durchgeführt wird.

**7.** Verfahren gemäß einem der Ansprüche 1 bis 6, **dadurch gekennzeichnet, dass** das Nitril ein aliphatisches Mono-, Di- oder Trinitril, ein cycloaliphatisches Mono- oder Dinitril, ein alpha-, beta- oder omega- Aminonitril oder ein Alkoxynitril ist.

**8.** Verfahren gemäß einem der Ansprüche 1 bis 7, **dadurch gekennzeichnet, dass**

i) N,N-Dimethylaminopropionitril (DMAPN) zur Herstellung von N,N-Dimethylaminopropylamin (DMAPA) eingesetzt wird, oder
ii) Isophoronnitrilimin zur Herstellung von Isophorondiamin eingesetzt wird, oder
iii) Adipodinitril (ADN) zur Herstellung von Hexamethylendiamin (HMD) oder zur Herstellung von 6-Aminocapronitril (6-ACN) und HMD eingesetzt wird.

**9.** Verfahren gemäß einem der Ansprüche 1 bis 8, **dadurch gekennzeichnet, dass** der Nickel-Festbettkatalysator einen Träger umfasst, vorzugsweise enthält der Träger ZrO$_2$.

**10.** Verfahren gemäß einem der Ansprüche 1 bis 9, **dadurch gekennzeichnet, dass** der Nickel-Festbettkatalysator als katalytisch aktive Masse die Komponente i) und gegebenenfalls die Komponente ii und/oder iii) umfasst, mit

i) Nickel (Ni)
ii) gegebenenfalls mindestens ein weiteres Element ausgewählt aus Eisen (Fe), Kobalt (Co), Ruthenium (Ru), Rhodium (Rh), Palladium (Pd), Osmium (Os), Iridium (Ir) oder Platin (Pt), und/oder
iii) gegebenenfalls mindestens einen Promotor ausgewählt aus Chrom (Cr), Mangan (Mn), Molybdän (Mo), Titan (Ti), Zink (Zn), Zinn (Sn), Alkalimetalle, Erdalkalimetalle, Seltene Erdmetalle oder Phosphor (P).

**11.** Verfahren gemäß einem der Ansprüche 1 bis 10, **dadurch gekennzeichnet, dass** der Nickel-Festbettkatalysator auf einem Katalysatorvorläufer beruht, dessen Zusammensetzung 40 bis 60 Gew.-% NiO, 10 bis 30 Gew.-% CuO, 1 bis 3 Gew.-% MoO$_3$ und 20 bis 40 Gew.-% ZrO$_2$ beträgt.

**12.** Verfahren gemäß einem der Ansprüche 1 bis 11, **dadurch gekennzeichnet, dass** ein Strom (S1) aus der Vorrichtung (V1) ausgeleitet wird, wobei der Strom (S1) das primäre Amin enthält und der Strom (S1) zumindest teilweise in die Vorrichtung (V1) rückgeleitet wird, vorzugsweise wird die Verbindung (I) zunächst in den rückgeleiteten Teil des Stromes (S1) zugegeben und als Bestandteil des Stroms (S1) in die Vorrichtung (V1) eingespeist, besonders bevorzugt wird die Verbindung (I) als wässrige Lösung in den rückgeleiteten Teil des Strom (S1) zugegeben.

**13.** Verfahren gemäß einem der Ansprüche 1 bis 12, **dadurch gekennzeichnet, dass** in der Vorrichtung (V1) unter Verwendung des gleichen Nickel-Festbettkatalysators wechselseitig eine Alkoholaminierung und eine Nitrilhydrierung durchgeführt wird.

**14.** Verfahren gemäß Anspruch 13, **dadurch gekennzeichnet, dass** das beim Wechseln von Nitrilhydrierung zur Alkoholaminierung oder beim umgekehrten Wechsel der Nickel-Festbettkatalysator nicht eingebaut und/oder ausgebaut werden muss.

15. Verfahren gemäß einem der Ansprüche 1 bis 14, **dadurch gekennzeichnet, dass** die Nitrilhydrierung kontinuierlich durchgeführt wird.

Europäisches
Patentamt

European
Patent Office

Office européen
des brevets

# EUROPÄISCHER RECHERCHENBERICHT

Nummer der Anmeldung

EP 15 15 6476

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2005 057198 A1 (BASF AG [DE]) 6. Juni 2007 (2007-06-06) * Absätze [0031] - [0033], [0057] - [0059], [0072] - [0073]; Ansprüche 1-34 * ----- | 1-15 | INV. C07C209/48 |
| A | WO 03/070688 A1 (CLARIANT GMBH [DE]; GOEBOELOES SANDOR [HU]; FASI ANDRAS [HU]; MARGITFA) 28. August 2003 (2003-08-28) * Ansprüche 1-34 * ----- | 1-15 | |

RECHERCHIERTE SACHGEBIETE (IPC)

C07C

Der vorliegende Recherchenbericht wurde für alle Patentansprüche erstellt

| Recherchenort | Abschlußdatum der Recherche | Prüfer |
|---|---|---|
| München | 29. Juli 2015 | Kleidernigg, Oliver |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

.....................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 15 6476

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-07-2015

| Im Recherchenbericht angeführtes Patentdokument | Datum der Veröffentlichung | Mitglied(er) der Patentfamilie | | Datum der Veröffentlichung |
|---|---|---|---|---|
| DE 102005057198 A1 | 06-06-2007 | KEINE | | |
| WO 03070688 A1 | 28-08-2003 | AT | 430122 T | 15-05-2009 |
| | | BR | 0307836 A | 07-12-2004 |
| | | CA | 2477085 A1 | 28-08-2003 |
| | | CN | 1635991 A | 06-07-2005 |
| | | DE | 10207926 A1 | 11-09-2003 |
| | | EP | 1480942 A1 | 01-12-2004 |
| | | ES | 2324161 T3 | 31-07-2009 |
| | | JP | 5264824 B2 | 14-08-2013 |
| | | JP | 5294368 B2 | 18-09-2013 |
| | | JP | 2005526041 A | 02-09-2005 |
| | | JP | 2010209092 A | 24-09-2010 |
| | | MX | PA04008134 A | 26-11-2004 |
| | | RU | 2292333 C2 | 27-01-2007 |
| | | US | 2005159624 A1 | 21-07-2005 |
| | | WO | 03070688 A1 | 28-08-2003 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 963975 A1
- EP 696572 B1
- WO 20111067199 A1
- EP 913388 A
- WO 2003070688 A
- EP 963975 A
- EP 1106600 A2

**In der Beschreibung aufgeführte Nicht-Patentliteratur**

- **TH. MASCHMEYER et al.** *Schema 9,* 1042
- **TH. MASCHMEYER et al.** The Reductive Amination of Aldehydes and Ketones and the Hydrogenation of Nitriles: Mechanistic Aspects and Selectivity Control. *Adv. Synth. Catal.,* 2002, vol. 344 (10), 1047
- Ullmann's Encyclopedia of Industrial Chemistry. 15. Juni 2000
- **A. B. STILES.** Catalyst Manufacture - Laboratory and Commercial Preparations. Marcel Dekker, 1983
- Catalysis and Catalysts. **VON ERTL et al.** Ullmann's Encyclopedia Electronic Release 2000. VCH, 1997, 28-32, 98
- **A. B. STILES.** Catalyst Manufacture. Marcel Dekker, Inc, 1983, 15
- Catalysis and Catalysts. **ERTL et al.** Ullmann's Encyclopedia Electronic Release 2000. VCH, 1997, 28-32, 98